# EUROPEAN PATENT SPECIFICATION

(11) **EP 2 709 639 B1**
(45) Date of publication and mention of the grant of the patent: **30.08.2017**
(21) Application number: 12727439.7
(22) Date of filing: 16.05.2012
(51) Int. Cl.: A61K 35/76, C12N 7/00

(54) **COMBINATION OF ONCOLYTIC ADENOVIRUSES WITH HISTONE DEACETYLASE INHIBITORS**
KOMBINATION AUS ONKOLYTISCHEN ADENOVIREN UND HISTONDEACETYLASE-HEMMERN
ASSOCIATION D'ADÉNOVIRUS ONCOLYTIQUES AVEC DES INHIBITEURS D'HISTONE DÉACÉTYLASES

(30) Priority: 16.05.2011 WO PCT/IB2011/052136
(43) Date of publication of application: 26.03.2014
(73) Proprietor: Institut Gustave Roussy, 94800 Villejuif (FR)
(72) Inventor: BRESSY, Christian, F-94230 Cachan (FR); GRELLIER, Elodie, F-94230 Cachan (FR); BENIHOUD, Karim, F-75013 Paris (FR)
(74) Representative: Cabinet Plasseraud
(86) International application number: PCT/IB2012/052469
(87) International publication number: WO 2012/156933

(56) References cited:
- FUEYO JUAN ET AL: "A mutant oncolytic adenovirus targeting the Rb pathway produces anti-glioma effect in vivo", ONCOGENE, vol. 19, no. 1, 6 January 2000 (2000-01-06), pages 2-12, XP002680362, ISSN: 0950-9232
- HOTI ET AL: "Valproic Acid, a Histone Deacetylase Inhibitor, Is an Antagonist for Oncolytic Adenoviral Gene Therapy", MOLECULAR THERAPY, vol. 14, no. 6, 18 November 2006 (2006-11-18), pages 768-778, XP005726580, ACADEMIC PRESS, SAN DIEGO, CA, US ISSN: 1525-0016, DOI: 10.1016/J.YMTHE.2006.07.009
- CINATL J ET AL: "SODIUM VALPROATE INHIBITS IN VIVO GROWTH OF HUMAN NEUROBLASTOMA CELLS", ANTI-CANCER DRUGS, vol. 8, no. 10, 1 November 1997 (1997-11-01), pages 958-963, XP000997406, LIPPINCOTT WILLIAMS & WILKINS, US; NL ISSN: 0959-4973, DOI: 10.1097/00001813-199711000-00007
- WATANABE T ET AL: "Histone deacetylase inhibitor FR901228 enhances the antitumor effect of telomerase-specific replication-selective adenoviral agent OBP-301 in human lung cancer cells", EXPERIMENTAL CELL RESEARCH, vol. 312, no. 3, 1 February 2006 (2006-02-01), pages 256-265, XP024944979, ACADEMIC PRESS, US ISSN: 0014-4827 [retrieved on 2006-02-01]
- DATABASE MEDLINE [Online] US NATIONAL LIBRARY OF MEDICINE (NLM), BETHESDA, MD, US; February 2012 (2012-02), KIM DAL RAE ET AL: "Combination therapy of conditionally replicating adenovirus and histone deacetylase inhibitors.", XP002680363, Database accession no. NLM22075951 & KIM DAL RAE ET AL: "Combination therapy of conditionally replicating adenovirus and histone deacetylase inhibitors.", INTERNATIONAL JOURNAL OF MOLECULAR MEDICINE, vol. 29, no. 2, February 2012 (2012-02), pages 218-224, ISSN: 1791-244X, DOI: 10.3892/IJMM.2011.831
- PARK MI-YOUNG ET AL: "Histone deacetylase inhibitors improve the antitumor effect of conditionally replicating adenovirus by increasing CAR expression.", PROCEEDINGS OF THE AMERICAN ASSOCIATION FOR CANCER RESEARCH ANNUAL MEETING, 14 April 2007 (2007-04-14), 18 April 2007 (2007-04-18), XP002680364, & 98TH ANNUAL MEETING OF THE AMERICAN-ASSOCIATION-FOR-CANCER-RESEARCH; LOS ANGELES, CA, USA ISSN: 0197-016X Retrieved from the Internet: URL:http://www.aacrmeetingabstracts.org/cg i/content/meeting_abstract/2007/1_Annual_M eeting/3300?maxtoshow=&hits=10&RESULTFORMA T=&author1=park&fulltext=histone&andorexac tfulltext=and&searchid=1&FIRSTINDEX=10&sor tspec=relevance&resourcetype=HWCIT [retrieved on 2012-07-20]

## Description

The invention relates to combination therapy in the field of cancer treatment, more specifically to the combination of oncolytic adenoviruses with histone deacetylase inhibitors (HDACis).

Oncolytic adenoviruses, also called conditionally replicative adenoviruses (CRAds) are mutant adenoviruses designed to replicate preferentially in tumour cells, killing them through cytopathic effect. Mainly two approaches have been used to create adenoviruses with tumour-cells specificity (for review see for instance: Chu et al., Clin Cancer Res, 10: 5299-5312, 2004; Yamamoto & Curiel, Mol. Therapy, 18, 2, 243-250, 2010):
(a) altering viral genes (generally E1A and/or E1B) essential for adenoviral replication in normal tissue but not in tumour cells;
(b) placing genes that initiate viral replication under the control of tumor specific promoters.

In spite of their potential interest as cancer therapeutics, the therapeutic efficiency of CRAds remains limited, mainly due to their difficulties to disseminate within solid tumors, thus requiring very high multiplicity of infection (MOI) to achieve effective killing of tumor cells.

Another promising approach for cancer therapy is the use of inhibitors of histone deacetylase.

Histone deacetylases (HDAC) are a class of enzymes that remove acetyl groups from N-acetylated lysines amino acid on histone proteins. Currently 18 HDACs have been identified in mammals. They have been divided into four classes based on cellular localization, function, and sequence similarity. Class I includes HDACs 1, 2, 3, and 8 which are found primarily in the nucleus. Class II HDACs (HDACs 4, 5, 6, 7 9, and 10) are found primarily in the cytoplasm but may be able to shuttle between the nucleus and the cytoplasm; class IIa comprises fours HDACs (HDACs 4, 5, 7 and 9) while class IIb comprises two HDACs (HDACs 6 and 10) which are expressed only in the cytoplasm. HDAC11, which is ubiquitously expressed, shares sequence similarities with both class I and class II HDACs and represents Class IV. Class III (also called "sirtuin family") groups NAD+-dependent proteins which do not act primarily on histones.

Based on their chemical structure, HDACis have been subdivided into four different classes (for review see for instance Dokmanovic & Marks, J. Cell. Biochem., 96, 293-304, 2005):
- hydroxamic acids (Trichostatin A, Vorinostat = SAHA)
- cyclic tetrapeptides (Depsipeptide)
- short-chain aliphatic acids (Valproic Acid, phenyl butyrate)
- benzamides (Entinostat).

These compounds are generally active (with depending on the compound, more or less specificity for a given HDAC) on histone deacetylases of Class I, II, and IV.

The inhibition of histone deacetylase by HDACi induces the accumulation of hyperacetylated nucleosome core histones. This chromatin remodeling results in modulation of gene expression, which can inhibit proliferation of cancer cells through biological processes such as cell cycle arrest, differentiation and/or apoptosis (for review see for instance Marks & Xu, J. Cell. Biochem., 107, 600-608, 2009; Federico & Bagella, J. Biomed. Biotechnol., 2011: 475641, 2011).

Table I below gives a list of some HDACi which are currently undergoing clinical trials.

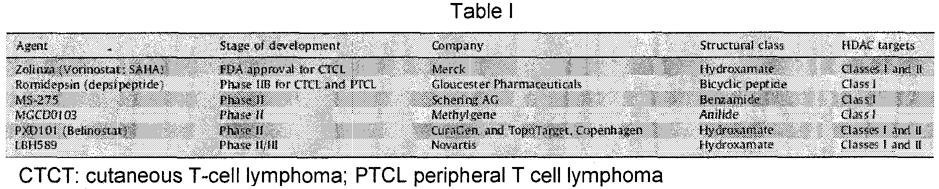

The inventors have now found that the combined treatment of tumor cells with an oncolytic adenovirus and an HDACi resulted in a significant reduction of the survival of these cells when compared with the treatments with the oncolytic adenovirus or with the HDACi alone.

They have also found that this effect occurs despite the fact that HDACi induces a reduction of the viral replication

The present invention is therefore providing a combination of:
- an oncolytic adenovirus ;
- a histone deacetylase inhibitor ;
for use as a medicament in cancer therapy as set out in claim 1. The invention to which this specification pertains is set out in claim 1 annexed to this description. According to a preferred embodiment of the invention, said oncolytic adenovirus has a mutation in the CR2 region of the adenoviral E1A, preventing its interaction with the host cell retinoblastoma protein (pRb), and its replication in normal cells which have a functional pRb, but not in tumour cells where the Rb pathway is defective. As a non-limitative examples of such oncolytic adenoviruses one can mention dl922-947 (Heise et al., Nat Med, 6, 1134-1139, 2000) also known as Δ24 (Fueyo et al., Oncogene, 19, 2-12 2000).

The oncolytic adenovirus and the HDACi can be administered in a single composition or separately. For instance, one or several administrations of the oncolytic adenovirus can be followed by a treatment with the HDACi, or conversely, the oncolytic adenovirus can be administered after a pretreatment with the HDACi. The best efficacy of treatment is obtained with injection of oncolytic adenovirus once a day for three days followed by daily injection of HDACi for 20 days.

The compositions of the present invention can be used for the treatment of various tumors. They are of particular interest in the treatment of tumours of colic, renal or ovarian origin.

### GENERAL MATERIALS AND METHODS:

### Cells

HT29, HCT116, SW480, SW620 colon adenocarcinomas cell lines, 911 human embryonic retinal cell lines and HEK293, a human embryonic kidney cell line expressing the adenoviral E1 protein were purchased from ATCC (American Tissue Culture Collection, Manassas, VA, USA).

HT29 and 911 were maintained in Dulbecco's modified Eagle's medium (DMEM;GIBCO) supplemented with 10% heat-inactivated fetal bovine serum (FBS;GIBCO).

The same medium was used for SW480 and SW620 supplemented with 1mM Sodium Pyruvate (Invitrogen/GIBCO)

HCT116 were cultured, in Mc Coy's 5A mediums with 10% heat-inactivated-FBS.

### Virus production

pXC1-Δ24 was a gift from Dr VW van Beusechem. pXC1-Δ24 is a plasmid derivated from pXC1 and contains a 24-nucleotides deletion, from Ad5 bp 923 to 946 (both included), corresponding to the amino acid sequence L₁₂₂TCHEAGF₁₂₉ of the E1A protein known to be necessary for Rb protein binding (Fueyo et al., 2000, cited above).

Mutation Δ24 was then cloned in the shuttle vector pXL3048 linearized by *Eco*RV. PXL3048 is a KmR-*Sac*B-ColE1 derivative (Crouzet et al., Proc. Natl. Acad. Sci. USA, 94, 1414-1419, 1997) containing the left end of the Ad5 genome (nucleotides 1-386), a polylinker with three unique cloning sites (*Eco*RV, *Bam*HI*,* and *Sal*I*),* and part of the Ad5 *pIX* gene (nucleotides 3446-4296). A homologous recombinaison was realized in *E. Coli* between PXL3048-Δ24 and *LacZ* recombinant control AdHwt (described as AE18 in Vigne et al., J Virol, 73, 5156-5161, 1999), which was derived from Ad5 with E1 and E3 regions deleted. After recombinational cloning in *E. coli,* the adenoviral genome was excised by *Pac*I digestion, and the CRAd Δ24 was recovered by transfecting 10 µg of *Pac*I-digested DNA into 293 cells by the Lipofectamine based procedure (Life Technologies, Inc.).

The CRAd Δ24 was propagated on HEK293 cells and purified with cesium chloride gradients. Then, virus were stored at -80°C in PBS 7% glycerol. The viral particles concentration was determined at 260 nm, and standard assay on 911 cells was performed to determine titers of infectious particles (plaque forming units; pfu)

### EXAMPLE 1: 1.REDUCTION OF COLON CARCINOMA CELLS SURVIVAL BY CO-TREATMENT WITH AN ONCOLYTIC ADENOVIRUS AND HISTONE DEACETYLASE INHIBITOR

A first series of experiments was performed on colon tumor cell lines HCT116, HT29, SW480, and SW620.

First, three different histone deacetylase inhibitors: Trichostatin A (TSA), an hydroxamic acid; Valproic Acid (VPA), a short-chain aliphatic acid, and sodium butyrate (Nabu), a short-chain aliphatic acid, were evaluated for their ability to induce inhibition of the growth of these cells after 48h of treatment using an MTT assay.

The results are shown in Table II below.

**Table II**

| Cell lines | HCT116 | HT29 | SW480 | SW620 |
|---|---|---|---|---|
| IC50 TSA (nM) | 35 | 150 | 75 | 37 |
| IC50 VPA (mM) | 1.2 | 10 | 10 | 5 |
| IC50 Nabu (mM) | 1 | 4 | 7 | 3 |

Then the cells were treated with one of these HDACis (at concentrations in the range of IC₅₀/2 and IC₅₀) and transduced with the oncolytic adenovirus Δ24, at increasing multiplicity of infection (MOI). The survival of the cells at day 3 p.i. was evaluated using the MTT assay.

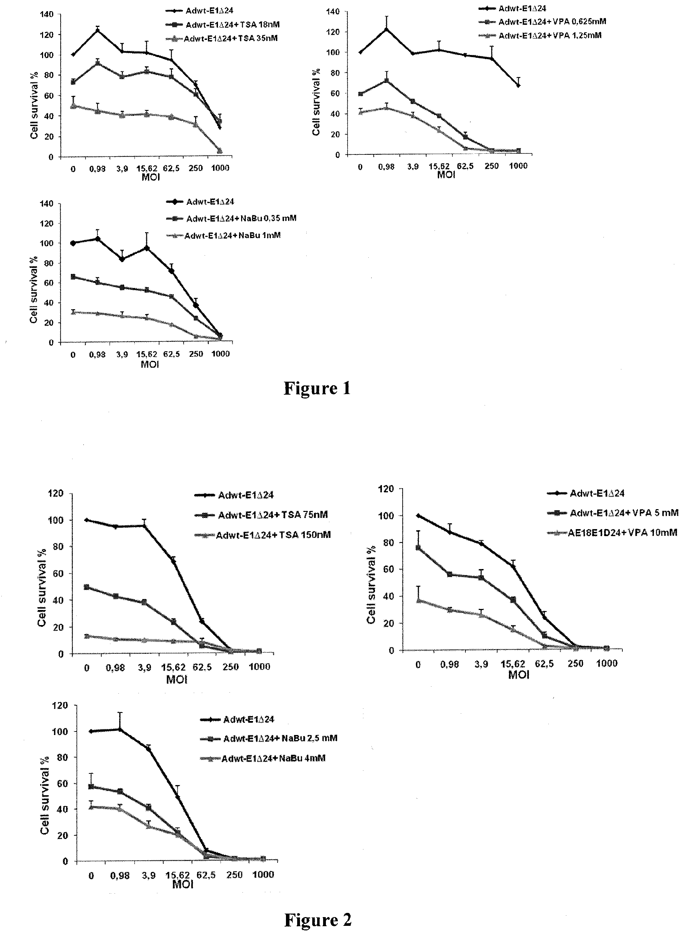

The results are shown on Figures 1 and 2 for HCT116 and HT29 cells respectively. The percent of survival of the cells at day 3 p.i. is represented as a function of the MOI for each concentration of HDACi.

It both cases a higher cytotoxicity is observed when the oncolytic adenovirus is combined with the HDACi. The same effects were observed in the case of SW480 and SW620 cells (results not shown).

In a second series of experiments, the effect of the combination of the oncolytic adenovirus with the HDACi VPA was more specifically studied and compared with the effect of the oncolytic adenovirus Δ24 (CRAd) alone or of the HDACi (VPA)alone.

Colon carcinoma cells (HT29, HCT116, SW480, SW620) were plated in quadruplicates on 96-well plates (10⁴ cells/well) and were treated with different MOI of Δ24 CRAd (ranging from 0 to 1000 vp/cell), VPA (0, IC25 or IC50) or a combination of CRAd and VPA. After 3 days, cell viability was measured with MTT assay and the results (means + SD) were expressed relative to non-infected cells treated with different VPA doses (0, IC25 and IC50). The results are representative of three experiments.

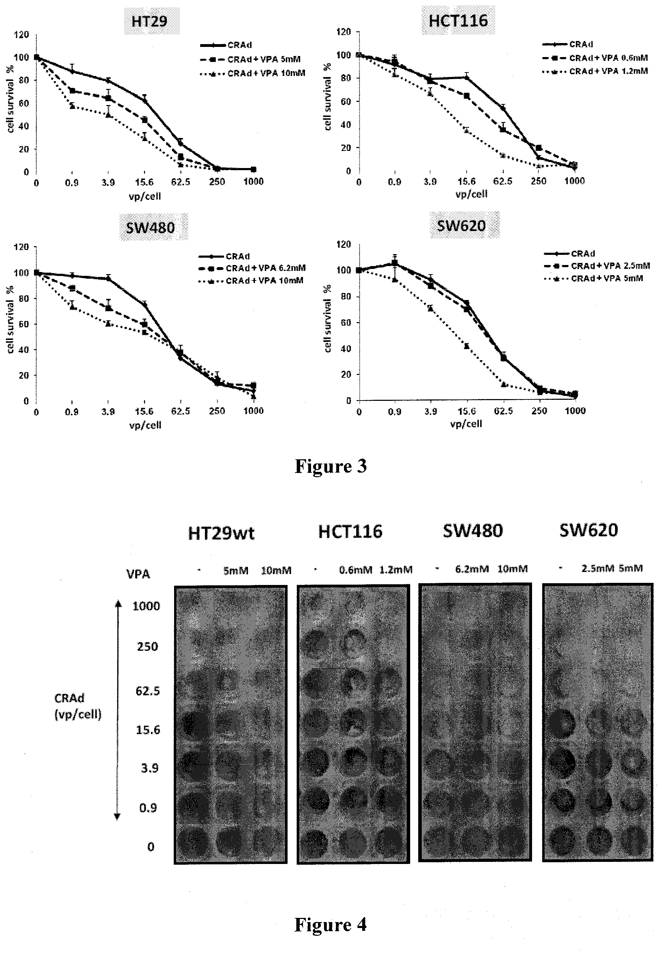

These results are shown on Figure 3.

For all cell lines, at three days post-infection, a reduction of cell survival was observed with increasing viral doses. Interestingly, HT29 cells treated with VPA (IC25) displayed a reduction in cell survival after infection with virus MOI of 0.9 to 250, this reduction was even more severe when increased VPA dose was used (IC50). In addition, a reduction in cell survival was demonstrated for the three other colon cell lines.

Cell survival was also evaluated using a crystal violet assay. Colon carcinoma cell lines were treated in triplicates with different MOI of Δ24 CRAd, VPA, or a combination of CRAd and VPA as described above. At day 3, the medium was removed and cells were stained with crystal violet 0.2% for 15 minutes. Then, plates were rinsed and dried before performing macroscopic observation.

The results are shown on Figure 4.

For HT29, SW480 and SW620, a reduction of cell survival was observed 72H post-infection with increasing doses of CRAd, HCT116 cells being less sensitive to the virus. Cells treated with CRAd and VPA displayed a dose-dependent reduction of cell survival compared to cells treated with CRAd or VPA alone. For example, VPA-treated HT29 cells infected at MOI ranging from 0.9 to 62.5 displayed a dramatic reduction in cell survival compared to HT29-only infected cells.

Time-dependent reduction of colon carcinoma cell survival after co-treatment with CRAd and VPA was also evaluated.

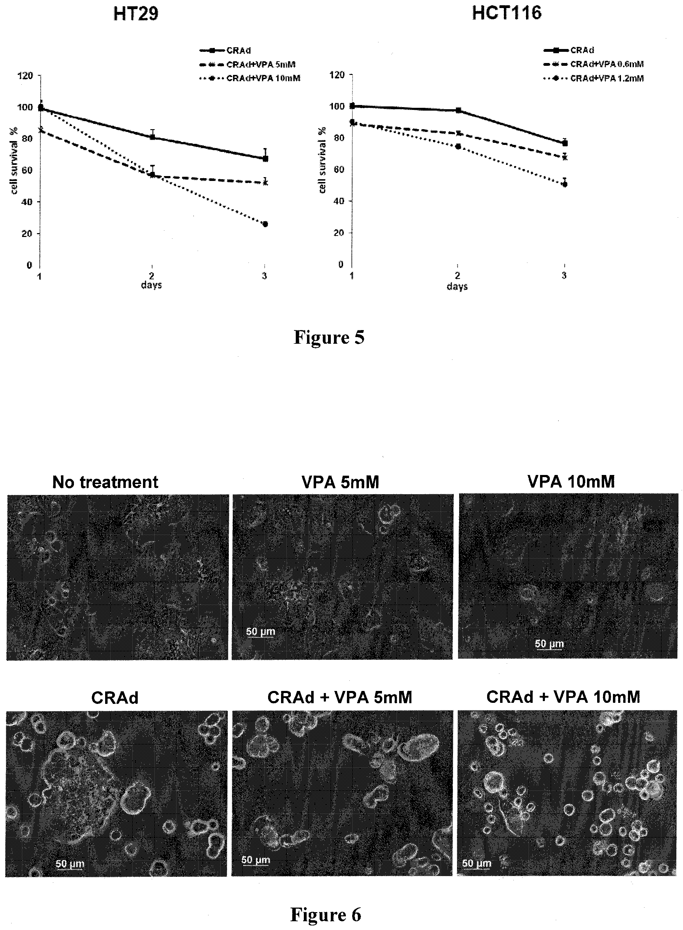

Colon carcinoma cells (HT29, HCT116) were treated in quadruplicates with Δ24 CRAd (MOI 15.6 vp/cell) without or with VPA (IC25 or IC50) and cell viability was measured at different time points thereafter using a MTT assay. The results (means + SD) were expressed relative to untreated or VPA (0, IC25 and IC50)-treated cells at the same time point. These results are shown on Figure 5. They are representative of two experiments.

When HT29 and HCT116 cells were infected by the virus (MOI 15.6), a slight reduction of cell survival was observed at day 2 and day 3 (until 20% reduction at day 3). Moreover, for both colon cell lines, a reduction of cell survival was observed at days 2 and 3 after CRAd+VPA co-treatment.

The effects of co-treatment with CRAd and VPA on morphology of colon cancer cells were also studied.

HT29 cells were untreated or treated with Δ24 CRAd (MOI 15.6) with or without VPA (IC25 or IC50). After 3 days, cells were observed by phase contrast microscopy. The results are shown on Figure 6 (Scale bar, 20 µm). They are representative of four experiments.

While non-treated HT29 cells appeared as a confluent monolayer, VPA-treated or CRAd-infected cells were less confluent. Interestingly, a dramatic decrease in the number of attached cells was observed for cells co-treated with CRAd and VPA.

The effects of co-treatment with CRAd and VPA on HT29 cell death were also evaluated by quantifying LDH release.

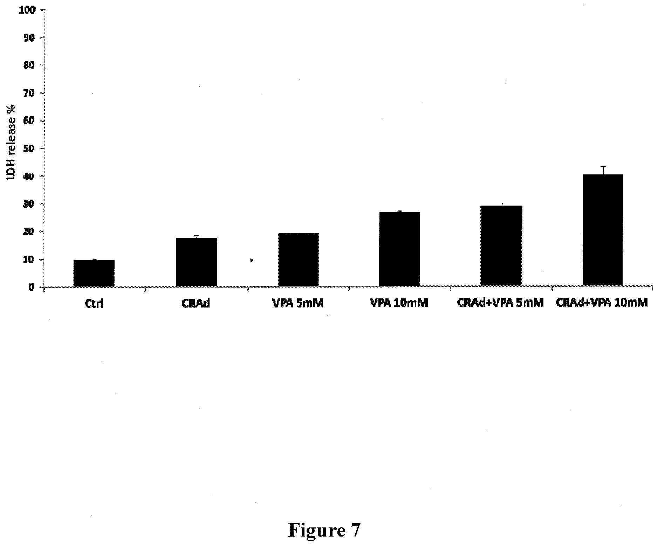

Colon carcinoma HT29 cells were untreated or treated with CRAd (MOI 15.6 vp/cell), VPA (IC25 or IC50) or both. After 3 days, cell death was quantified by determination of Lactate deshydrogenase (LDH) release in cell supernatant, using Tox Cytotoxicity Assay kit (TOX7, Sigma Aldrich, France). The results were expressed relative to total LDH level obtained with cells treated with identical conditions and permeabilized with triton 0.1%. The results (Mean + SD) are shown on Figure 7. They are representative of two experiments.

At day 3 CRAd induced a slight increase in LDH release compared to untreated cells. VPA triggered an increase in LDH release with higher effect observed for IC50 dose. Interestingly, cell co-treatment with CRAd and VPA led to up to 40% of LDH release. These results indicated that co-treatment with CRAd and VPA was able to kill HT29 cells more efficiently than single treatment with CRAd or VPA.

### EXAMPLE 2: CO-TREATMENT OF HT29 TUMORS XENOGRAFTED ON NUDE MICE WITH AN ONCOLYTIC ADENOVIRUS AND HISTONE DEACETYLASE INHIBITOR

Since CRAd and VPA were able to reduce cell survival *in vitro* and were shown to trigger cell death in colon carcinomas, we investigated their ability to modify *in vivo* the growth of tumor xenografts.

All animal experiments were approved by the IGR Institutional Animal Care and Use Committee.

HT29 cells (10⁷ cells) in 100µl PBS were injected subcutaneously into the right flank of athymic NU/NU female mice housed at the Institut Gustave Roussy.

First, the sensitivity of tumor xenografts to VPA treatment was evaluated. When tumors reached 70-100mm³, mice were injected intraperitoneally with VPA (200mg/kg or 300mg/kg) or PBS in a volume of 200µl five consecutive days per week. Tumor volume was evaluated twice a week for 4 weeks by measuring with calipers.

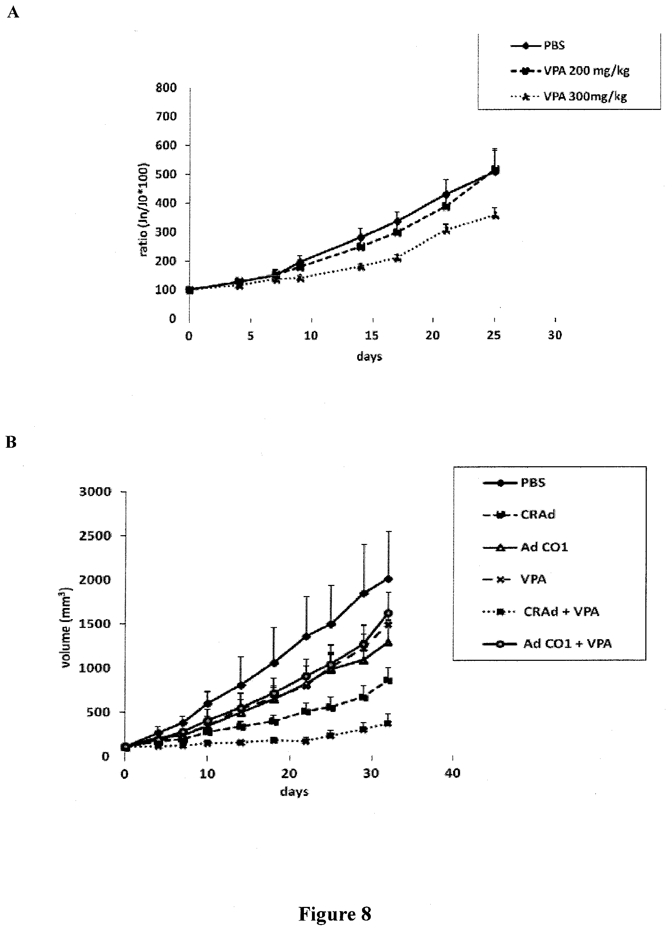

The results (means + SEM) are shown on Figure 8A. Tumor volume is expressed relative to the volume of the tumors at the beginning of the treatment.

No difference is observed in the kinetic of tumor growth between PBS or VPA (200mg/kg)-injected mice. In contrast, a significant delay in tumor growth was observed in mice injected with 300mg/kg of VPA. Of note, this VPA dose did not trigger significant toxicity.

In a second series of experimentation, when tumors reached 70-100 mm³, mice were first injected subcutaneously with Δ24 CRAd, or with a non-replicative adenovirus (AdCO1, deleted of region E1) or with PBS three consecutive days and every 7 days for 4 weeks. In parallel and beginning at day 4, mice received intraperitoneal injection of VPA (300mg/kg) or PBS in a volume of 200µl five consecutive days per week until the end of the protocol. Tumor growth was measured twice a week for 4 weeks.

The results (means + SEM) are shown on Figure 8B. Tumor volume is expressed in mm³.

When compared to PBS-injected mice, VPA- and AdCO1-treated mice displayed a reduction of tumor growth and CRAd-injected mice presented even a stronger reduction of tumor growth. Interestingly, treatment of mice with both CRAd and VPA provoked the strongest delay of tumor growth. This effect was not found with the combination of VPA and the replication-deficient virus AdCO1.

These results show that *in vivo* like *in vitro,* co-treatment with VPA and CRAd is more efficient to kill colon carcinoma cells than single treatment with VPA or CRAd.

### EXAMPLE 3: MECHANISMS OF TUMOR GROWTH INHIBITION BY CO-TREATMENT WITH AN ONCOLYTIC ADENOVIRUS AND AN HISTONE DEACETYLASE INHIBITOR

### 1) Effect of histone deacetylase inhibitor on viral replication

The effects of VPA on replication of CRAd were examined by monitoring the CRAd production, and the viral protein expression in HT29 cells transduced with CRAd and treated or not with VPA.

Colon cancer HT29 cells (5.10⁵) in 6-well plates were infected with Δ24 CRAd (MOI 15.6vp/cell) alone or in combination with VPA (IC50 and IC25) in adequate medium with 2% FBS. After 1 h, 3ml of growth medium with or without VPA was added. Then, cells were scrapped and cells and medium were harvested at different times post-infection (from day 1 to day 6). After submission of cells and medium to freeze-thaw cycles, the amount of infectious particles was determined by plaque assay on 911 cells and expressed as plaque forming unit (pfu)/ml.

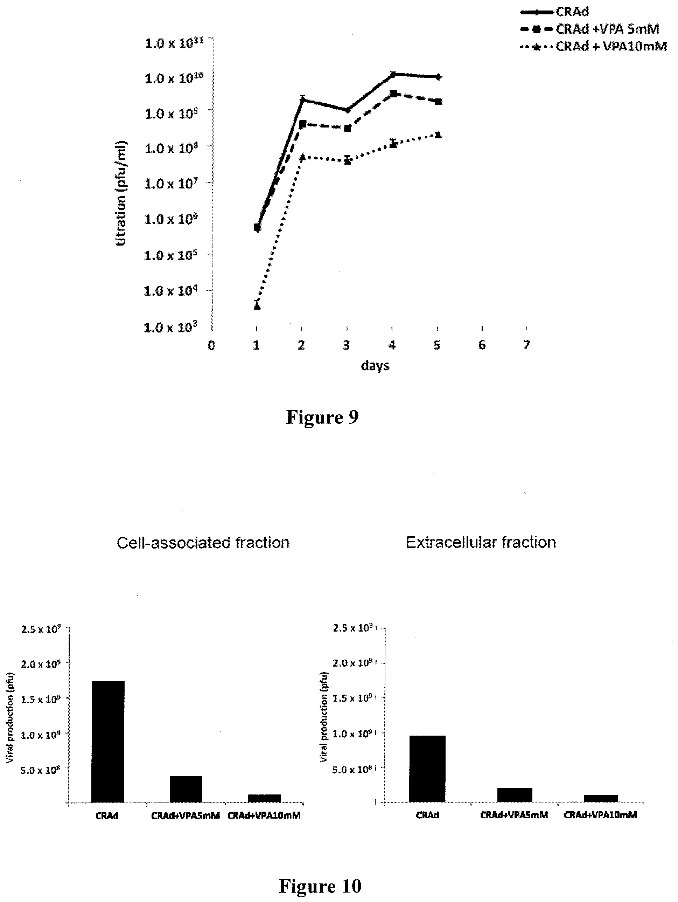

The results are shown on Figure 9. These results are representative of two experiments.

After HT29 treatment with CRAd, viral production increased from day 1 up to day 4. The co-treatment of this cell line with CRAd and VPA provoked a drop off of viral production at almost all time points analyzed. Moreover, viral production was severely impaired for IC50 VPA dose with a 10 to 100-reduction compared to CRAd only-infected cells.

In a second series of experimentations, HT29 cells (5.10⁵ cells/well) in 6-well plates were infected with CRAd (MOI 15.6vp/cell) with or without VPA (IC5O and IC25) in adequate medium supplemented with 2% FBS. After 1 h, 3 ml of growth medium with or without VPA was added. After 3 days, cells and medium were independently harvested and lysed by freeze-thaw cycles. Then, the amount of infectious particles was measured by plaque assay on 911 cells, and expressed as pfu/ml.

The results are shown on Figure 10. These results are representative of two experiments.

After 3 days, VPA induced a reduction of viral content in both cell-associated and extracellular factions.

For determining viral protein expression, HT29 cells (5.10⁵) in MW6 medium were treated with Δ24 CRAd (MOI 15.6 vp/cell), with or without VPA. At different time points p.i. (24h, 48h, and 72 h), tumor cells were harvested and protein extracts were prepared in RIPA buffer (10mM Tris base pH 8.8, NaCl 150mM, EDTA 1mM, NP-40 1%, deoxycholic acid 1%) supplemented with mini EDTA proteases (Roche laboratory). In order to quantify the expression of early (E1A) and late (fiber) adenoviral proteins, proteins (25 µg) were run into a NuPage (Invitrogen, France), transferred onto a nitrocellulose membrane, and probed with anti-fiber antibody (AB4 clone 4D2, THERMOSCIENTIFIC), anti-E1A antibody (sc-430, Santa Cruz), or (as a control) anti-actin antibody (Clone AC-15, SIGMA-ALDRICH) followed by HRP-conjugated secondary antibody.

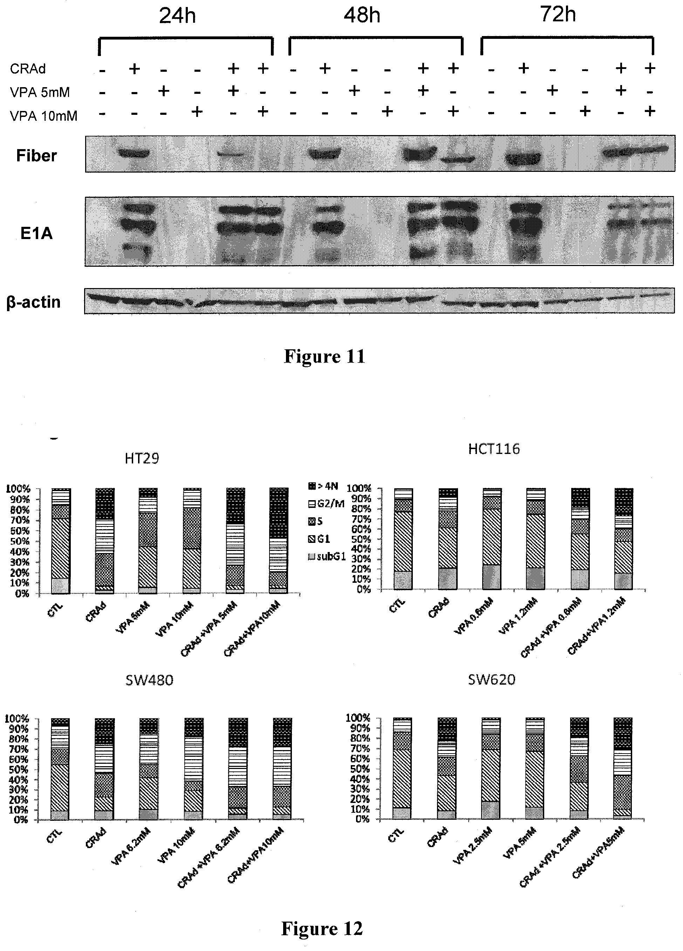

The results are shown on Figure 11. These results show that treatment of cell with VPA did not modify E1A expression, whatever the time point. In contrast, expression of the fiber protein was strongly diminished at day 1 for VPA-treated cells with a minor effect at day 2 and 3. In addition, the reduction of fiber expression at day 1 p.i. was more pronounced at the highest VPA dose. Altogether these results indicate that VPA did not modify early protein expression but delays the expression of late viral proteins.

The effects of VPA on CRAd replication were also tested *in vivo* in HT29 tumor xenografts of mice injected with PBS, Δ24 CRAd, or VPA (300mg/kg) + CRAd as described in Example 2 above. Tumors were excised at day 10, and expression of viral hexon protein was evaluated by immunohistochemistry. Tumors were fixed in FineFix (from IGR) paraffin-embedded, and cut into 4-µm-thick sections. Hematoxylin-eosin-safranin staining was performed on all of the xenografts for analysis of morphology. The polyclonal anti-adenovirus hexon protein antibody AB 1056 (CHEMICON INTERNATIONAL, Temecula, CA), diluted 1:300, was detected by a biotinylated rabbit antigoat immunoglobulin antibody streptavidin-horseradish peroxidase conjugate (DAKO), and the chromogen diaminobenzidine. Slides were counterstained with hematoxylin.

In the case of treatment by CRAd alone, a high amount of hexon protein is detected in the tumor. This amount is decreased by co-treatment with VPA.

Taken together, the above results show that the effects of the co-treatment do not stem from an increase of viral replication.

### 3) Effect of the co-treatment of the cell cycle

The effects of the co-treatment with Δ24 CRAd and VPA on the cell cycle in colon carcinoma cells (HT29, HCT116, SW480, SW620) were compared with those of the individual treatments with Δ24 CRAd or VPA.

Colon cancer cells (5.10⁵ cells/well) in 6-well plates were untreated or treated with CRAd (MOI 15.6 vp/cell) with or without VPA (IC25, IC50) for 72 hours. Then, cells were harvested, centrifuged and resuspended in sodium citrate buffer containing Triton X-100, RNAse 10 mg/ml and propidium iodide 1 mg/ml for 20 min at room temperature. Cell cycle distribution was determined by quantifying propidium iodide incorporation by flow cytometry analysis performed on FACSCALIBUR (BECKMAN).

The results are shown on Figure 12, which represents the percentage of sub-G1, G1, S, G2/M, and > 4n cells. For each cell line, these results are representative of two experiments.

For all cell lines, there is no increase of sub-G1 phase with CRAd, VPA or co-treatment with CRAd and VPA compared to untreated cells, ruling out a significant contribution of apoptosis in cell death. Virus infection with or without VPA provoked in all cell lines a reduction of G1 phase while VPA alone did not increase G1 phase. Interestingly, while untreated cells displayed a small population with DNA content superior to 4N, this population raised after CRAd infection and increased much more after co-treatment with CRAd and VPA. For example CRAd-infected HT29 cells exhibited 29.5% of cells with superior to 4N content versus 47.2% for CRAd-infected H29 cells co-treated with VPA and versus 1.3 for untreated cells.

### 4) Effect of the co-treatment on p21 expression

Colon cancer cells HT29 or HCT116 (5.10⁵) in MW6 were treated with Δ24 CRAd (MOI 15.6 vp/cell), with or without VPA (IC25 and IC50). At 24, 48, and 72h p.i., tumor cells were harvested and protein extracts were prepared in RIPA buffer (10mM Tris base pH 8.8, NaCl 150mM, EDTA 1mM, NP-40 1%, deoxycholic acid 1%) supplemented with mini EDTA proteases (ROCHE LABORATORY). Proteins (25 µg) were run into a NuPage (INVITROGEN, France), transferred onto a nitrocellulose membrane, and probed with anti-p21/Waf1 mouse monoclonal antibody (EA10, CALBIOCHEM) and anti-actin antibody (AC-15, SIGMA-ALDRICH), followed by HRP-conjugated secondary antibody. p21 expression was quantified by IMAGE-J software and expressed as arbitrary units relative to untreated cells.

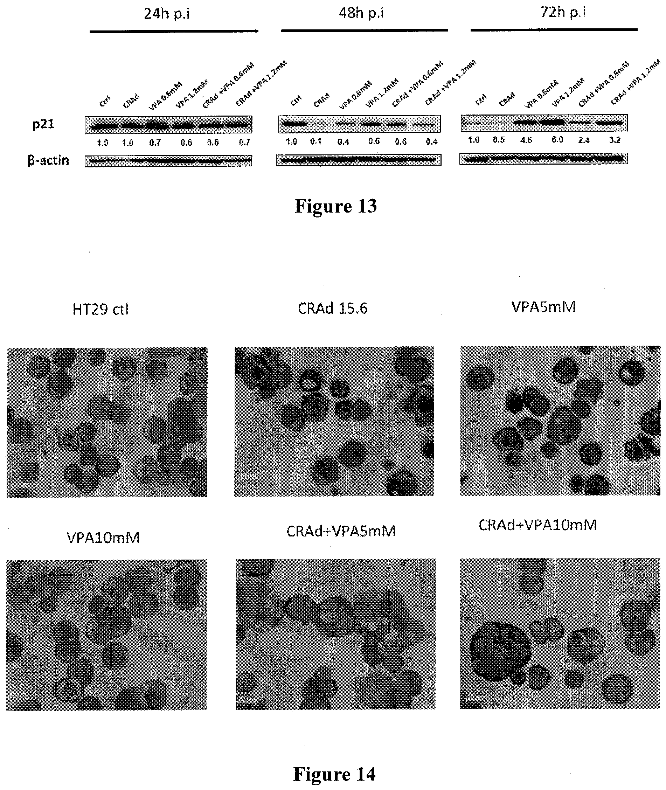

The results for HT116 are shown on Figure 13. The numbers indicate the level of p21 relative to untreated cells.

No expression of p21 was observed in HT29 cells in agreement with previous published data. Therefore, we documented p21 expression in HCT116, another colon carcinoma cell line. P21 was detectable in untreated HCT116 cells with a reduction of its expression at 48h and 72h. In contrast VPA led to a dramatic increase in p21 expression (up to 6-fold compared to untreated cells). HCT116 co-treated with CRAd and VPA presented an intermediate expression pattern of expression between those obtained with CRAd alone and VPA alone.

### 5) Effect of the co-treatment on cell size and nucleus morphology

HT29 cells in 6-well plates (5.10⁵ cells/well) were untreated or treated with Δ24 CRAd (MOI 15.6 vp/cell), VPA (IC25, IC50) or both. After 72h, cells were harvested, cytospined on slides, fixed with Wright and stained for 15 minutes in Giemsa solution. After washing, dry slides were mounted with a coverslip. Cells and nuclei observations were performed using phase-contrast microscopy.

The results are shown on Figure 14.

Compared to untreated cells, HT29 cells treated with VPA exhibited an increase in cell size and cell nuclei with well-defined shape. Cell infected with CRAd without VPA did not display significant change in cell size and nucleus morphology. In sharp contrast, a significant proportion of HT29 cells that have undergone co-treatment with CRAd and VPA displayed an increase in cell size with irregular nucleus morphology or several nuclei.

### 6) Effect of the co-treatment on γH2AX expression

Colon carcinoma HT29 and HCT116 cells (5.10⁵) in MW6 were treated with Δ24 CRAd (MOI 15.6 vp/cell), with or without VPA (IC25 and IC50). At indicated time points p.i., the cells were harvested and protein extracts were prepared in RIPA buffer (10mM Tris base pH 8.8, NaCl 150mM, EDTA 1mM, NP-40 1%, deoxycholic acid 1%) supplemented with mini EDTA proteases (ROCHE LABORATORY). Protein extracts were sonicated and centrifuged. Then, protein extracts (25 µg) were run into a NuPage (INVITROGEN, France), transferred onto a nitrocellulose membrane, and probed with anti-phospho-H2AX (Ser139) antibody (clone JBW301, MILLIPORE) or anti-actin antibodies (AC-15, SIGMA-ALDRICH) followed by HRP-conjugated secondary antibody. γH2AX was quantified by IMAGE-J software and expressed as arbitrary units relative to untreated cells.

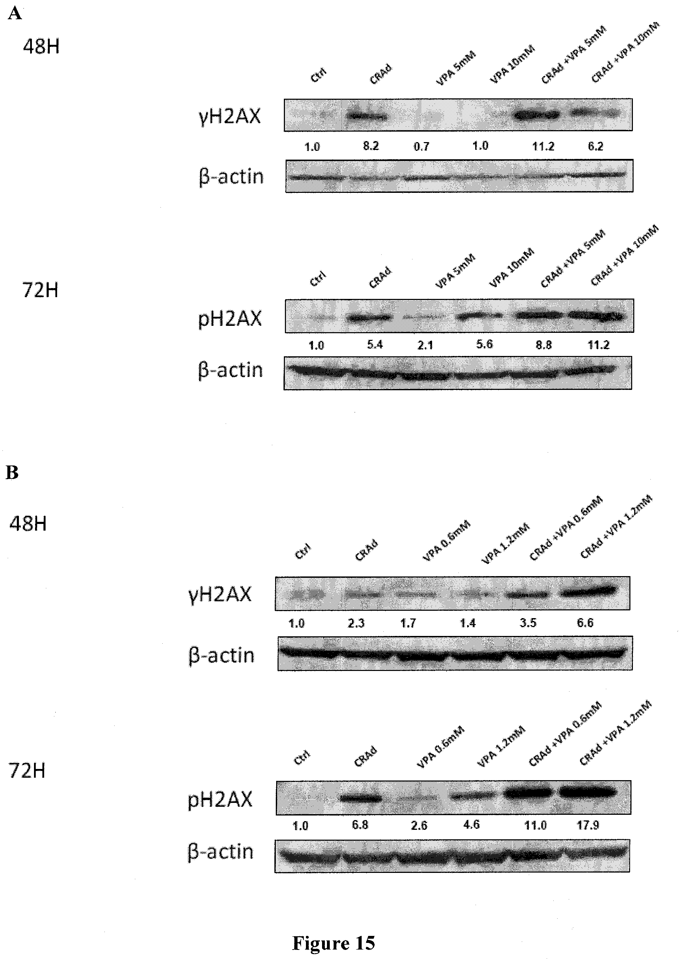

The results are shown on Figure 15 (A: HT29; B: HCT116). The numbers indicate the level of γH2AX relative to untreated cells.

CRAd is able to strongly induce γH2AX at day 2 and 3 in both HT29 and HCT116. In addition, increase in γH2AX was also observed after 3 days in VPA-treated HT29 and HCT116 cells but to a lesser extent than CRAd. Most interestingly, treatment with CRAd and VPA together led to a dramatic increase in γH2AX expression in the two cell lines and at the two time points examined. For example, the level of γH2AX was increased 11.2-fold after co-treatment with CRAd and VPA 10mM compared to 5.4 and 5.6 after treatment with CRAd or VPA 10mM, respectively.

Also, γH2AX expression was analyzed in HT29 cells by confocal microscopy.

HT29 cells were seeded at 5.10⁵ cells/well on 6-well plates and cultured overnight. Cells were treated with Δ24 CRAd (MOI 15.6 vp/cell), VPA (IC25, IC50) or both (CRAd+VPA), or with the non-replicative adenovirus AE18 during 72h. Then non-adherent tumor cells and adherent tumor cells were detached with TrypLE™ Express (1X) (INVITROGEN) cytospined on slides (500 rpm, 5 min), washed with PBS and fixed with 4% paraformaldehyde for 30 min. After permeabilization with 0.5% Triton X-100 for 10 min, samples were blocked with 3% BSA for 30 min. Samples were exposed to primary antibodies (1:1,000 anti- yH2AX, for 1-2 h in PBS/BSA) followed by Alexa Fluor-conjugated secondary antibody staining for 1 h. When filamentous actin was also stained, tetramethyl-rhodamine B isothiocyanate-labeled phalloidin (SIGMA) was added with the secondary antibody incubation. Cells were washed four times with PBS after secondary antibody staining and then mounted on glass slides by using mounting medium with DAPI (VECTASHIELD; Vector Laboratories). All images were obtained by using the ZEISS LSM 510 confocal microscope.

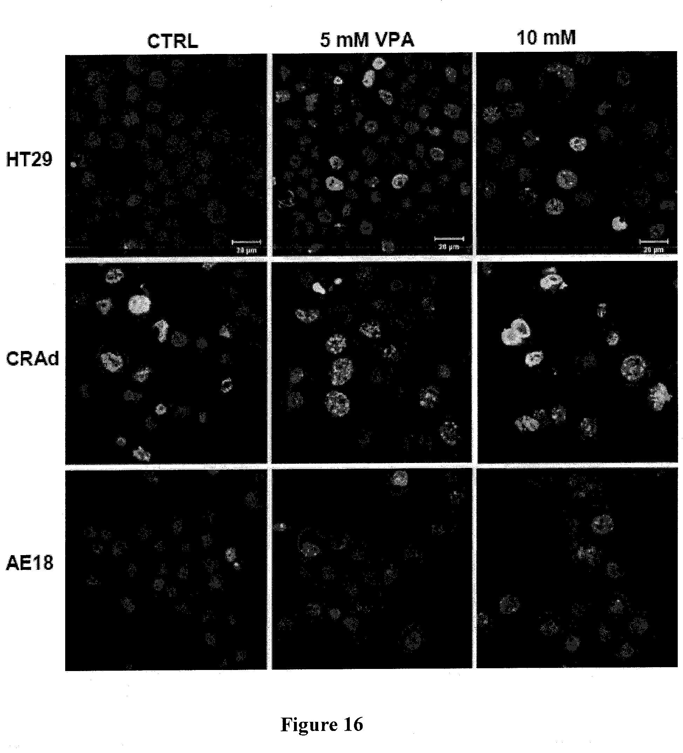

The results are shown on Figure 16.

For HT29 cell lines, Δ24 CRAd alone induces a high γH2AX expression at 3 days. VPA is also able to induce γH2AX protein but much less than CRAd. Besides, AE18, a recombinant Ad deficient for replication can induce a very low level of γH2AX in same way as VPA alone. Interestingly, CRAd + VPA have shown the highest increase of γH2AX expression compared to CRAd or VPA alone or recombinant Ad AE18 with or without VPA.

### 7) Reduction in tumour cell survival requires E1 expression

The effects of a co-treatment with the oncolytic adenovirus Δ24 and the histone deacetylase inhibitor VPA on the survival of HT29 cells 3 days p.i. were compared with those of a co-treatment with VPA and the recombinant adenovirus AE18. The experiments were performed as disclosed in Example 1.

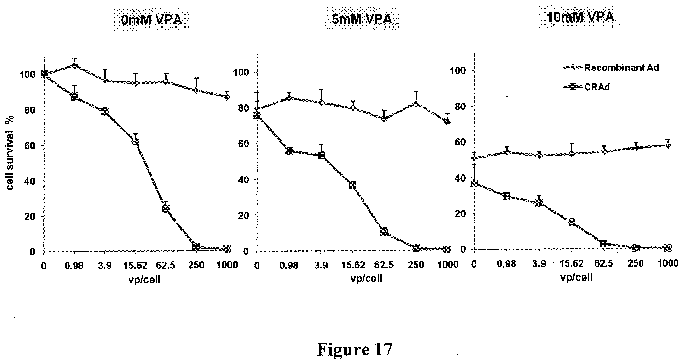

The results are shown on Figure 17. These results show that VPA has no effect on the cytotoxicity of the recombinant adenovirus AE18.

## Claims

1. A combination of:
- an oncolytic adenovirus consisting of an adenovirus delta24 ; and
- an histone deacetylase inhibitor consisting of valproic acid ;
for use as a medicament in cancer therapy.

## Patentansprüche

1. Kombination aus:
- einem onkolytischen Adenovirus, bestehend aus einem Adenovirus delta 24; und
- einem Histondeacteylase Inhibitor, bestehend aus Valproinsäure;
zur Verwendung als Medikament zur Krebstherapie.

## Revendications

1. Combinaison de :
- un adénovirus oncolytique constitué d'un adénovirus delta24 ; et
- un inhibiteur d'histone désacétylase constitué de l'acide valproïque ;
pour une utilisation en tant que médicament dans le traitement du cancer.
